# EUROPEAN PATENT SPECIFICATION

(11) **EP 1 744 685 B1**
(45) Date of publication and mention of the grant of the patent: **17.10.2012**
(21) Application number: 05741669.5
(22) Date of filing: 27.04.2005
(51) Int. Cl.: A61B 17/34

(54) **SEALS FOR TROCARS**
DICHTUNG FÜR TROKARE
JOINTS POUR TROCARTS

(30) Priority: 05.05.2004 US 839776
(43) Date of publication of application: 24.01.2007
(73) Proprietor: ConMed Corporation, Utica, NY 13502 (US)
(72) Inventor: PIECHOWICZ, Michael, E., Marcy, NY 13403 (US); WING, Daniel, M., Utica, NY 13502 (US)
(74) Representative: Vuillermoz, Bruno
(86) International application number: PCT/US2005/014393
(87) International publication number: WO 2005/112799

(56) References cited:
- WO-A-02/30305
- WO-A-02/41795
- US-A- 5 342 316
- US-A- 5 865 807
- US-A1- 2002 013 552
- US-A1- 2003 040 711
- US-A1- 2004 260 244

## Description

### TECHNICAL FIELD

This invention relates generally to surgical instruments, and more for particularly to seals for trocar systems for providing an opening through tissue and into body cavities and through which surgical instruments may be inserted.

### BACKGROUND OF THE INVENTION

Trocar systems are surgical devices used to obtain access to a body cavity to perform various surgical procedures such as laparoscopic surgery or arthroscopic surgery.

A trocar system typically includes a pointed rod-like device or obturator fitted into a tube-like device or cannula. The cannula head of the cannula often has one or more seals. A pointed end of the obturator projects out an end of a cannula tube and is used to penetrate the outer tissue of the cavity. Alter the tissue is penetrated and the body cavity is accessed by the trocar system, the obturator is then withdrawn while the cannula tube is retained in the cavity. The body cavity can then be accessed by surgical instruments via the cannula tube to perform various surgical procedures, or the cannula can simply be used as a drainage outlet.

The document WO 02/41795 discloses a seal according to the preamble of claim 1. However, there is still a need to further improve seals for trocar systems which allow a surgeon to provide an opening in a cavity of a patient and though which surgical instruments may be inserted.

### SUMMARY OF THE INVENTION

The present invention provides a seal with a seal according to claim 1.

In yet another aspect, the present invention provides a method for sealing a cannula head with a seal according to claim 1.

In further aspects, the present invention provides cannula heads and trocar systems that include the above-described seals.

### BRIEF DESCRIPTION OF THE DRAWINGS

The subject matter which is regarded as the invention is particularly pointed out and distinctly claimed in the concluding portion of the specification. The invention, however, may best be understood by reference to the following detailed descriptions of the various embodiments and the accompanying drawings in which:

FIG. 1 is a perspective view of a dilating trocar system in accordance with the present invention;

FIG. 2 is a perspective view of a cutting trocar system in accordance with the present invention;

FIG. 3 is a side elevational view, in part cross-section, of the dilating trocar system of FIG. 1 showing the obturator removed from the cannula;

FIG. 4 is a side elevational view, in part cross-section, of the cutting trocar system of FIG. 2 showing the obturator removed from the cannula;

FIG. 5 is an enlarged, exploded, cross-sectional view of the cannula of FIGS. 1 and 2 and a releasably attachable upper seal in accordance with the present invention;

FIG. 6 is an enlarged, exploded, cross-sectional view of the cannula head of FIGS. 1 and 2 showing a lower seal with a flapper valve open;

FIG. 7 is an exploded, perspective view of the lower seal of FIG. 6;

FIG. 8 is an exploded, cross-sectional view of the lower seal of FIG. 6;

FIG. 9 is an enlarged perspective view, partially cutaway, of the releasably attachable upper seal of FIG. 5;

FIG. 10 is a top view of the protective covering guide of the releasably attachable upper seal shown in FIG. 9;

FIG. 11 is a cross-sectional view of the releasably attachable upper seal of FIG. 9;

FIG. 12 is a cross-sectional view of another embodiment of a releasably attachable upper seal in accordance with the present invention;

FIG. 13 is a cross-sectional view of another embodiment of a releasably attachable upper seal in accordance with the present invention;

FIG. 14 is a top view of a cannula head with a releasably attachable upper seal shown in dashed lines in an unlocked position;

FIG. 15 is a top view of a cannula head with a releasably attachable upper seal shown in a locked position;

FIG. 16 is a cross-sectional view of a releasably attachable upper seal with an obturator extending therethrough; and

FIG. 17 is a cross-sectional view of a releasably attachable upper seal with an obturator off centered extending therethrough.

### DETAILED DESCRIPTION OF THE INVENTION

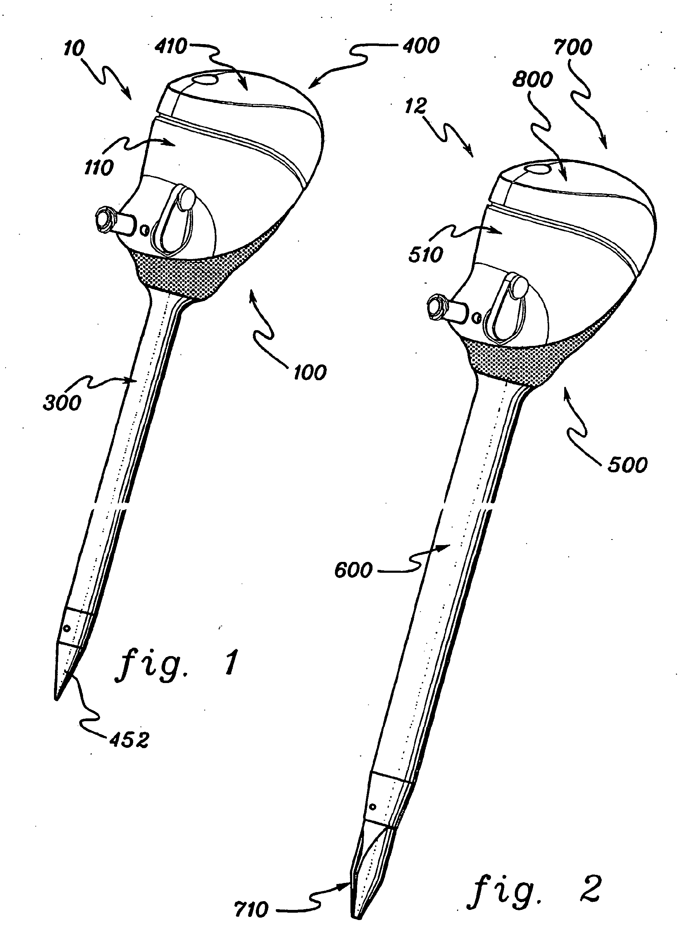

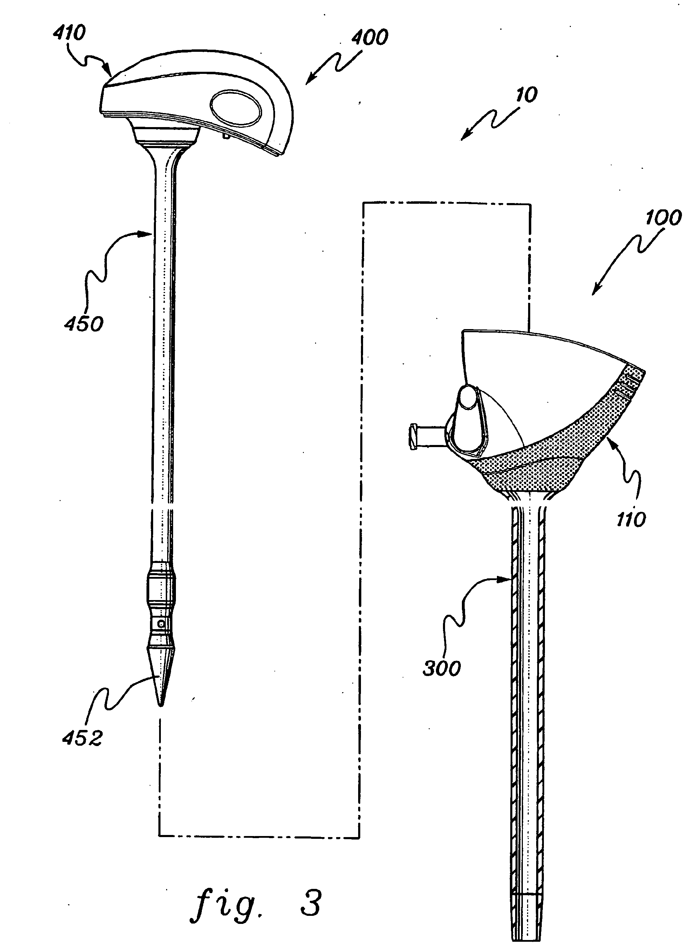

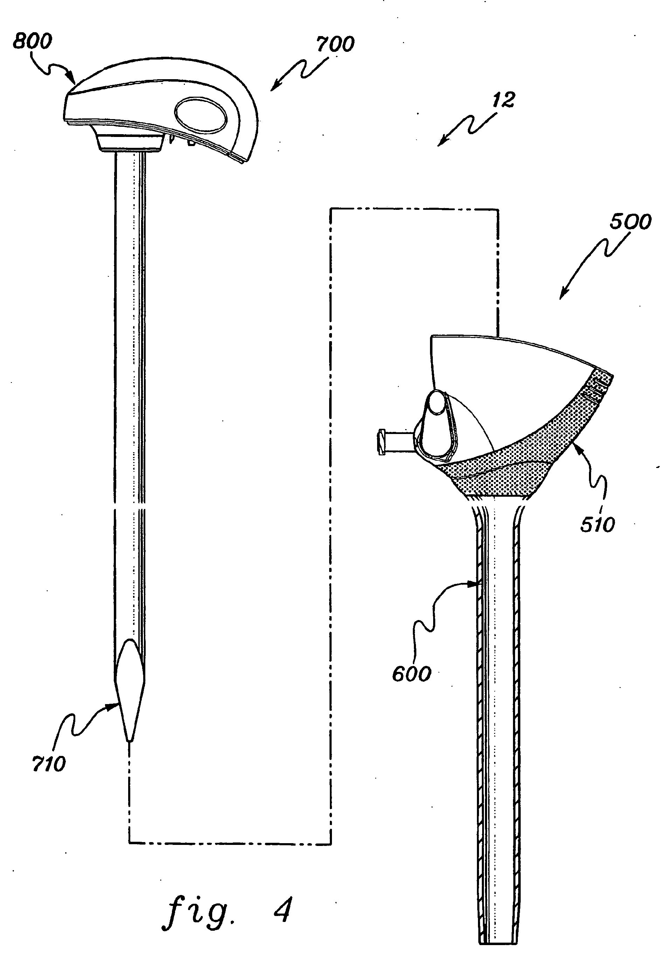

FIGS. 1 and 2 illustrate perspective views of a dilating trocar system 10 and a safety-shielded cutting trocar system 12, respectively, which may incorporate a seal in accordance with the present invention as explained in greater detail below. With reference to FIGS. 1 and 3, dilating trocar system 10 generally includes a cannula 100 having a cannula head 110 and a cannula tube 300 into which is slidably receivable a dilating obturator 400 having an obturator cap 410 attached to an elongated shaft 450 (FIG. 3) having a pointed end 452 which may have a rounded point at its distal end. With reference to FIGS. 2 and 4, trocar system 12 generally includes a cannula 500 having a cannula head 510 and a cannula tube 600 into which is slidably receivable a safety shielded cutting obturator 700 having a cap 800 and a spring-loaded shield 710 and automatically-operated spring-loaded locking mechanism for inhibiting the exposure of a cutting blade (not shown) after passing through tissue or muscle. The cutting trocar system cuts or lacerates tissue when obtaining access to a body cavity. The dilating trocar systems allow parting and stretching of, for example, multiple cross-directional muscle layers and intra-abdominal blood vessels, when gaining access to a body cavity.

The above-noted trocar systems may include one or more of the various features described in U.S. Patent Application No. US 2003/060770*.*

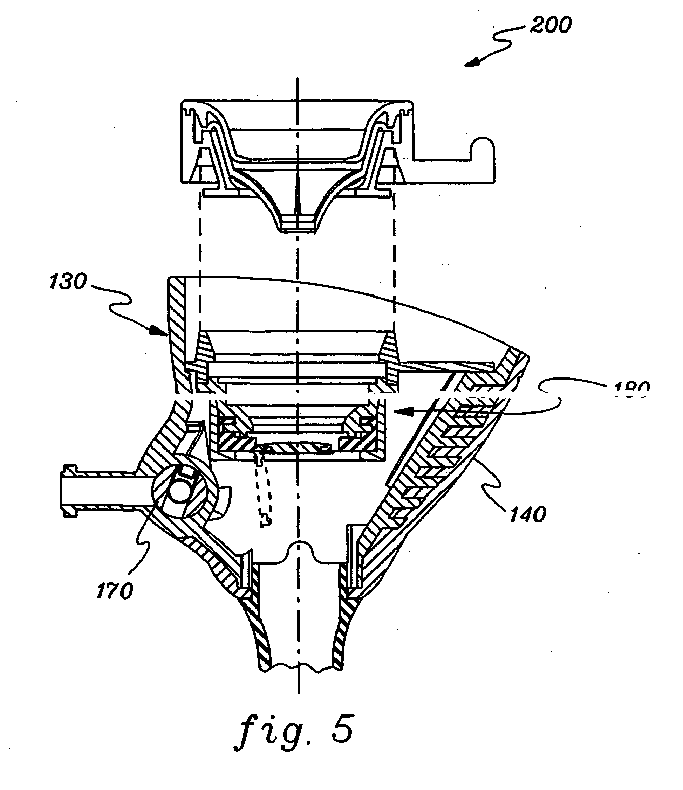

As shown in FIG. 5, the cannulas may include a cannula housing 130, resilient non-slip material 140, a releasably attachable stopcock valve 170, a lower seal 180, and a releasably attachable upper seal 200.

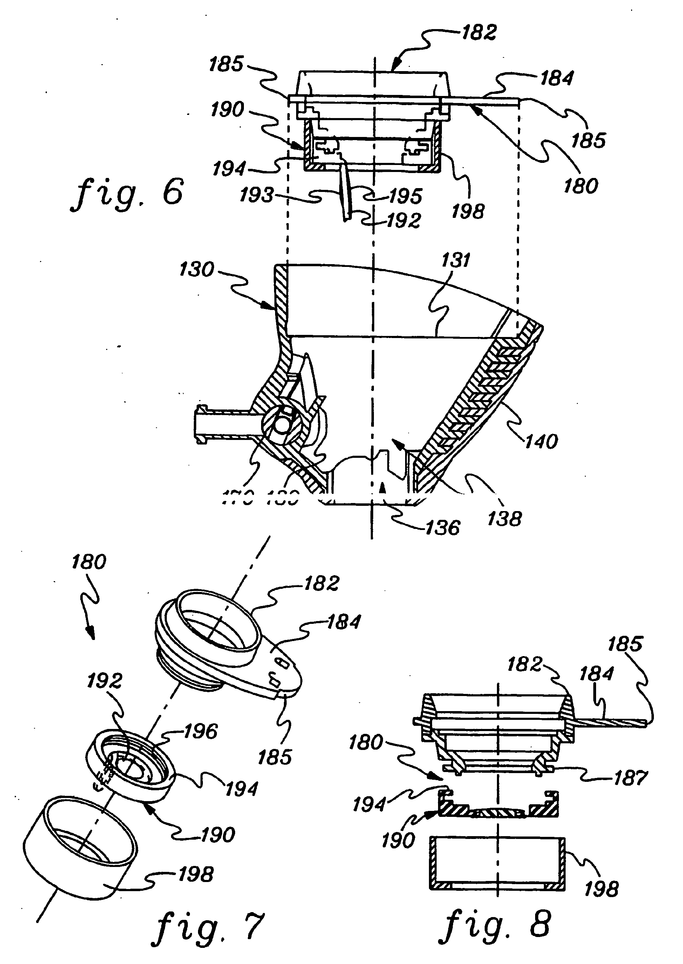

As best shown in FIGS. 6-8, lower seal 180 includes an outer support 182, a flapper valve 190, and a retainer cap 198. Lower seal 180 is sealably attached across passageway 136 (FIG. 6) in cannula housing 130 (FIG. 6) behind stopcock valve 170 (best shown in FIG. 5). For example, an outer edge 185 of a lateral flange 184 may be attached with an adhesive (or other suitable means for forming a complete seal) along a support or ledge 131 (FIG. 6) formed in cannula housing 130 (FIG. 6) to define a chamber 138 (FIG. 6) in housing 130 (FIG. 6).

Flapper valve 190 includes a flexible disc-shaped portion 192 (shown in an open position in FIG. 6) attached at a portion along its circumference to a flexible collar 194 having a groove 196 (FIG. 7) therein. A pair of rigid discs 193 and 195 (best shown in FIG. 6) may be attached to the center of flexible disc-shaped portion 192. The rigid discs may add support to and protect the flexible disc-shaped portion when an obturator or other instruments are inserted and removed from the cannula. Outer support 182 may be monolithic or integrally formed as one-piece. Outer support 182 may also include a circumferentially extending flange 187 (best shown in FIG. 8) on which is received flexible collar 194 of flapper valve 190. When no instrument is inserted in the cannula, the flapper valve 190 is normally closed, i.e., biased shut. In addition, if the cannula housing is pressurized with a fluid, the pressurized fluid will exert a pressure against the flapper valve to more securely retain the flapper valve in a sealed position and maintain the pressure in the cannula housing and in the body cavity. Retainer cap 198 further secures and maintain flapper valve on outer support 182. In particular, retainer cap 198 aids in retaining collar 194 (FIG. 6) or flapper valve 190 (FIG. 6) on outer support 182 (FIG. 6) when an obturator is inserted in the cannula. Retainer cap may include one or more notches or cutouts (not shown) on the lower portion thereof adjacent to where the flapper valve attaches to flexible collar 194. This notch or notches allow the hinge of the flapper valve to be more flexible and seat properly if caught by a hooked instrument as it is withdrawn from the cannula.

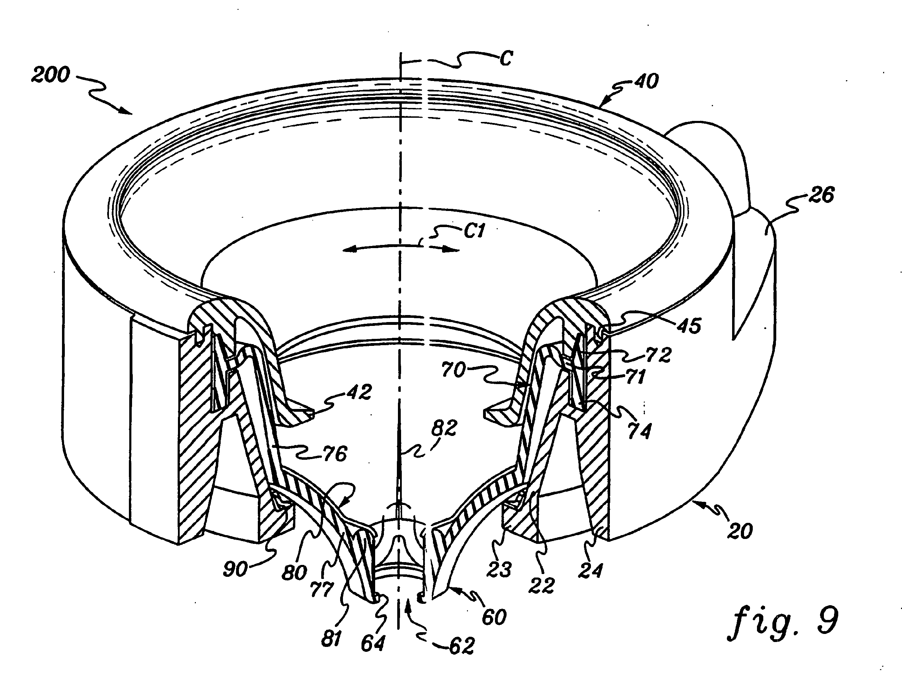

As shown in FIG. 9, releasably attachable upper seal 200 may include a rigid distal support 20, a rigid proximal support 40, a rotatable flexible seal 60 having upper portions sandwiched between the supports, a movable ring 90 disposed between the flexible seal and the lower support, and a generally non-compliant, but bendable protective guide 80. As described above, upper seal assembly 200 is releasably and sealably attachable to the cannula head of the trocar as illustrated in FIG. 5.

With reference again to FIG. 9, distal support 20 includes an inner ring-shaped portion 22 having an inwardly-extending flange 23 defining a distal opening, an outer ring-shaped portion 24, and an outwardly-extending arm 26. Outwardly extending arm 26 allows a surgeon to readily lock and unlock upper seal assembly 200 to a cannula head, e.g., via a bayonet-style connector as described in greater detail below. This locking and unlocking arrangement permits a surgeon to remove upper seal 200 if it becomes necessary during surgery to withdraw a tissue sample or another sizeable item from the surgical site. Otherwise, upper seal 200 generally remains in place during a surgical procedure because it can accept instruments having various diameters.

Rigid proximal support 40 includes an inwardly-extending flange 42 which defines a proximal opening. The openings in distal support 20 and proximal support 40 guide and limit lateral movement of an instrument inserted in upper seal assembly 200. Proximal support 40 may also have a concave shape for directing a tip of an instrument through the upper seal. The distal and proximal supports may be made from a rigid plastic material.

Rotatable flexible seal 60 includes a distal centrally located aperture or opening 62 which allows for easy insertion and removal, for example, of an instrument such as an obturator shaft into a cannula head while inhibiting the release of fluid from the cannula head. The distal end of rotatable flexible seal 60 may also include and inwardly extending flange 64 which assists in conforming and sealing against relatively small instruments but which flattens out against and conforms to relatively large instruments. The distal most portion of rotatable flexible seal 60 also includes a raised sinusoidal portion 81 that extends around the inner, lower portion of the upper seal to define a plurality of inwardly extending lobes. This configuration allows the opening to easily expand and conform around relatively large instruments.

Rotatable flexible seal 60 further includes an upper seal portion 70 having a horizontally-extending portion 71, an upwardly-extending annular flange 72, and a downwardly-depending annular flange 74. The flanges 72 and 74 and a portion of the horizontally-extending portion 71 are fitted between portions of proximal support 40 and an annular groove in distal support 20. An outer downwardly-depending flange 45 of proximal support 40 may be received in and fixedly attached, e.g., with adhesive or by ultrasonic welding, to an annular groove in distal support 20.

Upper seal portion 70 of the rotatable flexible seal is sized slightly smaller than the corresponding cutout formed in the rigid supports. Accordingly, this allows the seal to rotate when an instrument is inserted in and twisted about the central axis C of the seal in the direction of curved double-headed arrow C1. In addition, the pressurized cannula results in forcing the outer surface of downwardly-depending annular flange 74 against the inside surface of the cutout formed in rigid support 20 to form an airtight seal to seal pressurized gas in the cannula head and prevent leakage.

Disposed between upper seal portion 70 and opening 62 is a generally downwardly-depending vertical frusta conical wall portion 76 and a convex wall portion 77. Rotatable flexible seal 60 may be made from a resilient or an elastomeric material, for example, a silicone rubber, a polyurethane elastomer, a neoprene or a thermoplastic elastomer. A lubricant may be applied to the outer surface of the upper seal 70 (e.g., to the upper flange portion) or to the entire surface of the upper seal 70. The lubricant may be a coating, such as parylene.

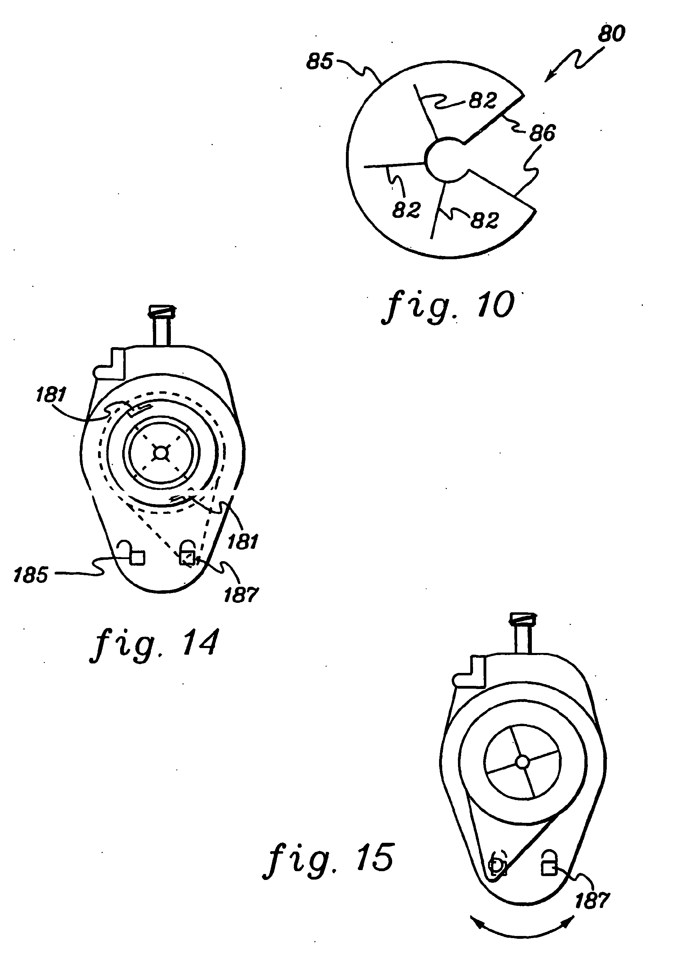

Protective guide or liner 80 is attached, for example with an adhesive, along an inner surface of convex seal portion 77. A lower portion of the protective guide is spaced apart from or disposed away from opening 62 in rotatable flexible seal 60 in the vicinity of the lobes, short of orifice 62, thus leaving a portion of seal 60 exposed for making a seal with an instrument. Protective guide further includes four slits 82, only one of which is shown in FIG. 9. As best shown in FIG. 10, protective guide 80 may be fabricated from a flat sheet initially in the form of an arc such as from a slippery, bendable material such as a thin layer or sheet of plastic. Slits 82 are formed extending from an inner portion 84 to a point adjacent to an outer edge of an outer portion 85. Accordingly, when protective guide 80 is attached to the inner surface of convex portion 77 (FIG. 9), edges 86 are disposed adjacent to each other. The slits in the guide permit the opening in the guide to expand radially outward as the width of each slit widens upon insertion of an instrument.

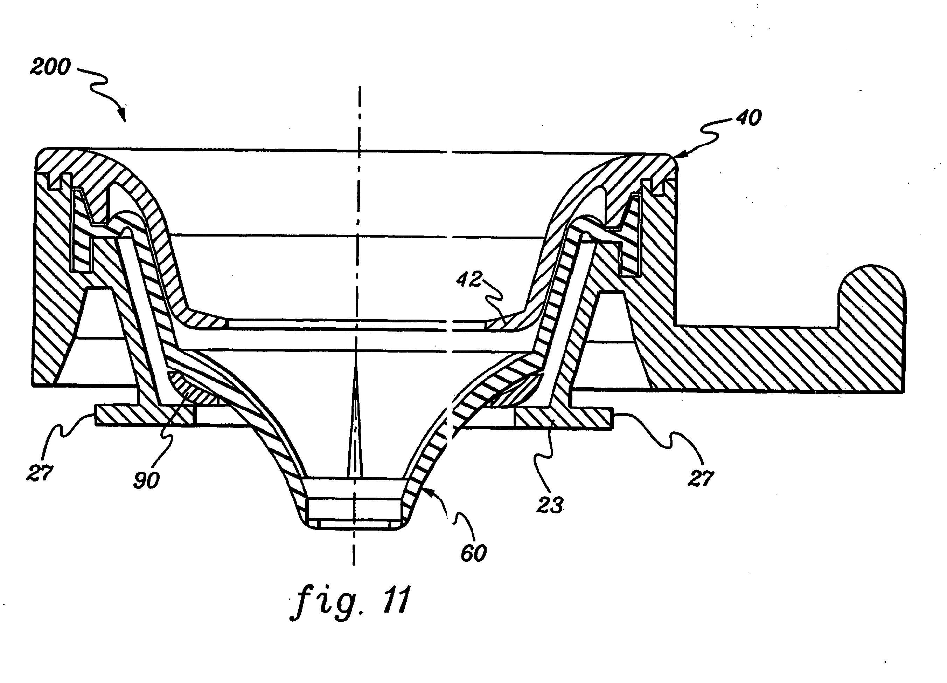

As best shown in FIG. 11, a movable washer 90 may be disposed between rotatable flexible seal 60 and flange 23. The purpose of washer 90 is to restrain the distal movement of flexible seal 60 when an instrument of large diameter is inserted therein and to inhibit the rotatable flexible seal 60 from being stretched distally to such an extent that it becomes jammed at the orifice of the lower flapper valve. Movable washer 90 may be made to generally replicate the shape and the dimensions of flange 42 of proximal support 40. For example, the washer may have a curved cross-section. Thus, the central opening in washer 90 may essentially match that of the central opening of flange 42. Washer 90 may be fabricated from a rigid plastic material such as Acrylonitrile Butadiene Styrene (ABS), polycarbonate, nylon, etc.

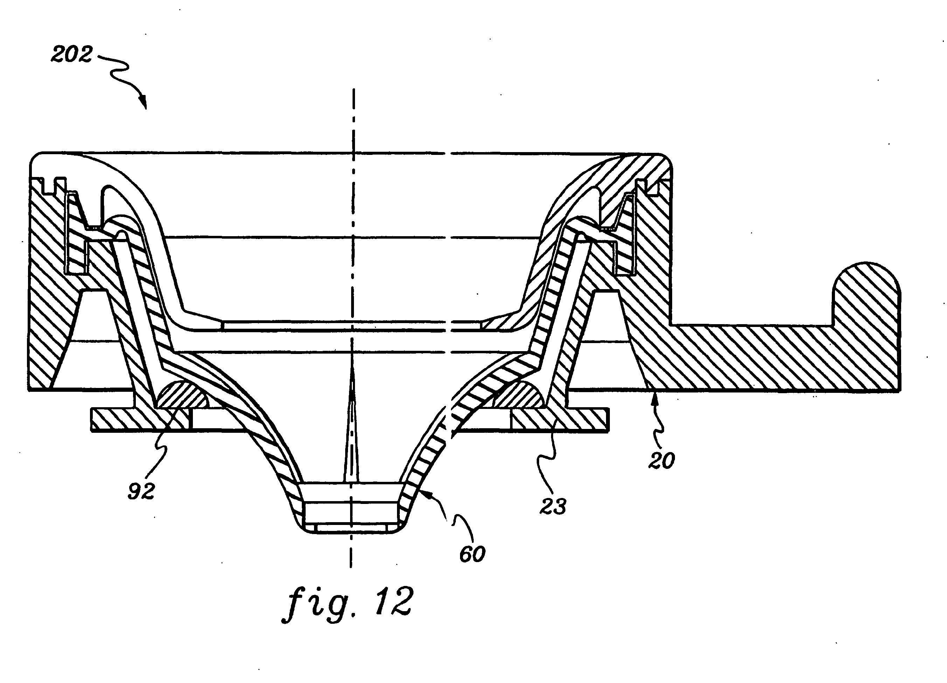

FIG. 12 illustrates another embodiment of releasably attachable upper seal 202 in accordance with the present invention. In this embodiment, a ring 92 configured as half of an O-ring (e.g., having a half round cross-section) may be disposed between rotatable flexible seal 60 and flange 23. Ring 92 may be formed from a resilient material such as silicone rubber or other suitable material, and may be fixedly attached to flange 23 with an adhesive.

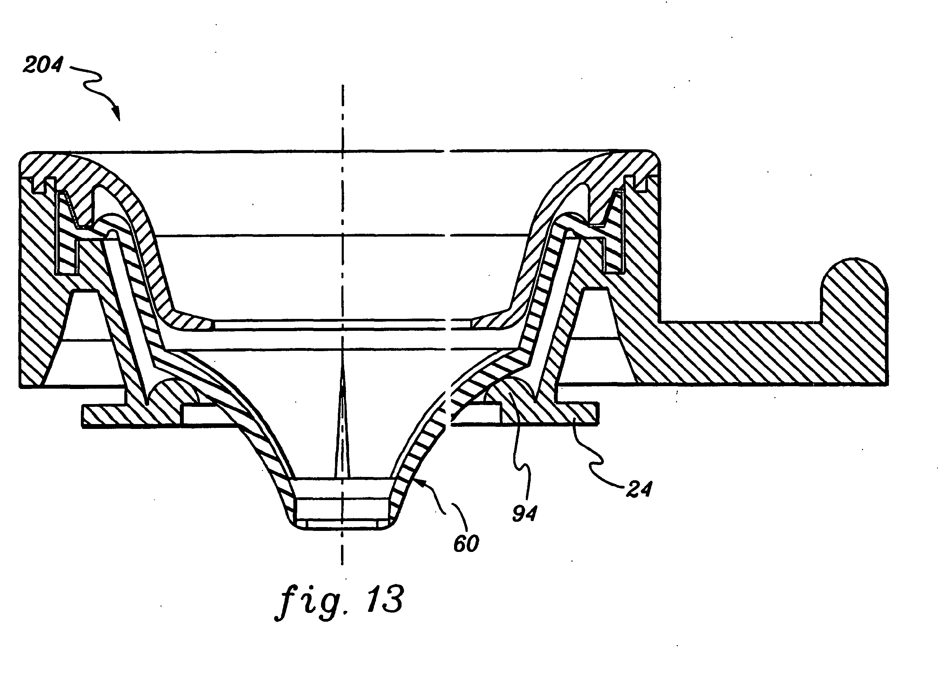

FIG. 13 illustrates another embodiment of releasably attachable upper seal 204 in accordance with the present invention. In this embodiment, a flange 24 of rigid distal support 20 is configured to include a raised portion 94 which extends upwardly and inwardly toward flexible seal 60. Thus, raised portion 94 may be integrally formed as a unit with distal support, i.e., being monolithic or as one piece.

For example, the proximal opening defined by flange 42 may measure about 1/2 of an inch in diameter and the orifice in the seal may measure about 1/8 of an inch in diameter. Flange 23 on distal support 20 may define a distal opening measuring about 5/8 of an inch in diameter. The washer opening may measure about 1/2 of an inch in diameter.

As shown in FIG. 14, a releasable attachable seal is shown in dashed lines in an unlocked position. As shown in FIG. 15, the releasably attachable seal is releasably attached to the cannula head by rotating the releasably attachable seal clockwise.

The cannula head and the releasably attachable seal may be releasably attachable, for example, via a bayonet-type connector, with the releasably attachable seal having two flexible pins or tabs 27 (FIG. 11) that extend outwardly and engage J-shaped grooves 181 (FIG. 14) in the lower seal of the cannula head. It will be appreciated that other means for releasably attaching the upper seal to the cannula head may be employed.

In addition, indicia 185 and 187 may be provided on the lower seal for indicating the locked and unlocked positions of the releasably attachable upper seal. By providing a releasably attachable upper seal, a surgeon may remove the releasably attachable upper seal to provide a greater opening for removing, for example, tissue through the cannula tube and cannula head. The aperture of the upper seal may be sized so that the aperture or hole is slightly smaller than an obturator shaft or surgical instrument to be used. This interference fit between the resilient diameter of the aperture and the shaft minimizes the passage of fluid from a cavity to the ambient environment during insertion and removal of an obturator or other instruments. When an obturator, for example, is removed from the cannula and from the aperture, the flapper valve provides a seal to the ambient environment.

From the present description, it will be appreciated that the cannula head may have a fixedly attached upper seal. In addition, the releasably attachable upper seal may include a stationary ring or fixedly attached ring while the flexible seal is operable to rotate within the supports. In addition, the releasably upper seal may include a nonrotatable flexible seal and a movable or fixedly attached ring. It will also be appreciated that the ring may have other suitable cross-sectional configurations.

It will be appreciated by those skilled in the art that the diameter of the opening in the proximal support, the thickness of the flexible seal, and the diameter of the aperture in the flexible seal, may vary depending upon the size of the cannula head, the size of the obturator or other instruments to be used, and the difference in pressure across the flexible seal that needs to be sealed. For example, the thickness of the flexible seal may be between about 1 mm to about 3 mm, and the thickness of the protective guide may be about 0,254 mm to about 0,76 mm (0.010 inches to about 0.030 inches).

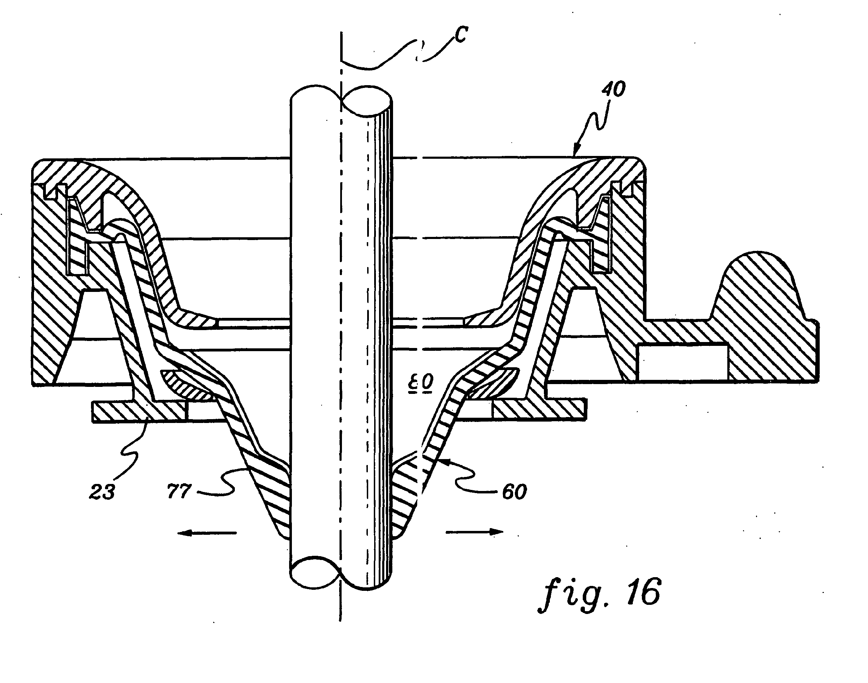

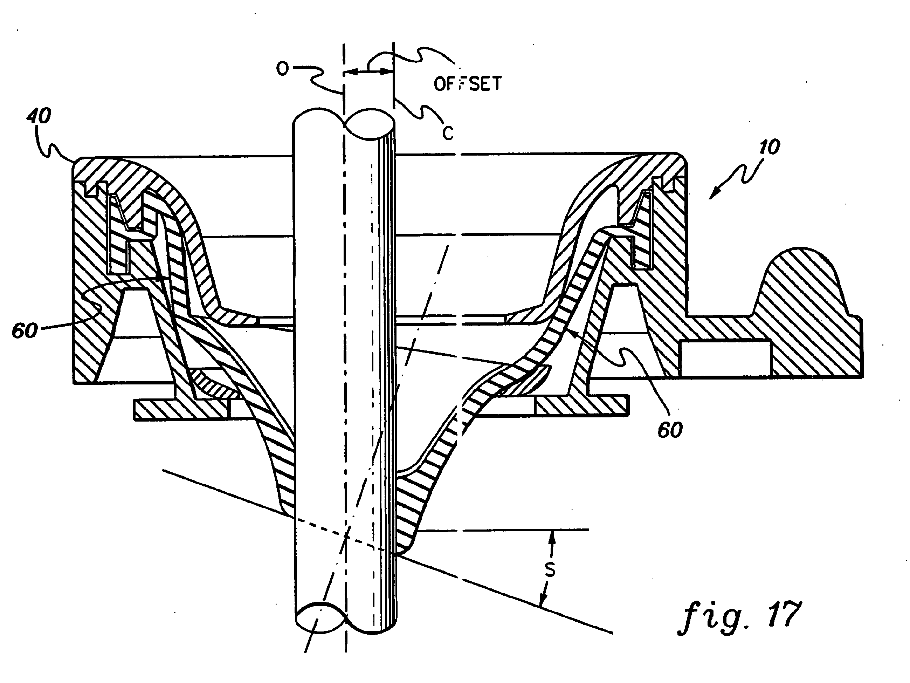

With reference to FIGS. 16 and 17, when assembled, the releasably attachable upper seal is designed primarily so that any lateral movement of the seal 60 will be rather limited when an instrument is initially inserted off axis. Instead, the instrument is guided or deflected toward the central axis C. In particular, the combination the dish-shaped proximal support 40, the funnel or conical shape of rotatable flexible seal 60, the conical shape and slipperiness of the protective guide 80 which extends generally over the portion of the flexible seal exposed under the proximal opening formed in the proximal support 40, the washer, and the lower flange, all result in a distal point of an instrument being guided or deflected toward the central opening of the flexible seal.

As shown in FIG. 16, an instrument such as a shaft of an obturator is ideally inserted along central axis C of the releasably attachable upper seal. As the point of an obturator or instrument is inserted, the opening of the seal 60 expands in the direction of the horizontal arrows to form a seal around its shaft. Under normal circumstances, an instrument inserted off axis will simply be deflected by protective guide 80 toward the central axis C.

As best shown in FIG. 17, if a relatively large force is applied and maintained to hold the off axis (e.g., along axis O) once the shaft of the obturator is fully inserted in the releasably attachable upper seal, the combination of flexible seal 60 and protective guide 80 deforms and tilts resulting in the lower portion thereof maintaining a seal around the shaft.

In particular, the upper portion of flexible seal 60 closest to the shaft is deformed and pushed upwardly against proximal support 40, and the upper portion of flexible seal 60 farthest away from the shaft is pulled downwardly. In addition, the combination of the flexible seal 60 and protective guide 80 are deformed and disposed on an angle to central axis C and the lowermost portion of flexible seal 60 is deformed and disposed on an angle S around shaft 100. The lowermost portion-forming the resulting opening in flexible seal 60 around the shaft is no longer circular but instead oval shaped.

## Claims

1. A seal (200, 202, 204) for a cannula head (110, 510), said seal (200, 202, 204) comprising:
- a support (20, 40) defining a passageway extending therethrough and defining an axis;
- a flexible seal (60) rotatably supported by said support (20, 40) across said passageway, said flexible seal (60) comprising an upper portion (70) having a horizontally-extending portion (71), and at least one of an upwardly-extending annular flange (72) and a downwardly-depending annular flange (74); wherein
said support (20, 40) comprises a cutout for receiving said upper portion (70) of said flexible seal (60), said flanges (72, 74) and a portion of said horizontally extending portion (71) being disposed in said cutout and spaced-apart from surfaces of said cutout so that
lateral movement of said flanges (72, 74) limited;
**characterized in that** said flexible seal (60) is rotatably supported by said support (20, 40) is so that the flexible seal is rotatable only around said axis.

2. The seal of claim 1, wherein said seal (200, 202, 204) comprises a guard (80) having a plurality of slits for protecting said flexible seal (60), and wherein said flexible seal (60) comprises a lubricant coating.

3. The seal of claim 1, wherein said seal (200, 202, 204) is releasably attachable to the cannula head (110, 510).

4. The seal of claim 1, wherein at least one of said support (20, 40) comprises an arm (26) outwardly extending from said support.

5. The seal of claim 4, wherein said arm (26) comprises an upwardly-extending knob.

6. The seal of claim 1, wherein it further comprises a ring (90, 92, 94) disposed between a lower portion of said support (20, 40) and said flexible seal (60), and wherein said ring (90, 92, 94) defines an opening sized smaller than an opening in said lower portion of said support (20, 40).

7. The seal of claim 6, wherein said ring (90, 92) is movable between said flexible seal (60) and said lower portion of said support (20, 40).

8. The seal of claim 6, wherein said ring (90, 92) is movably positioned between said flexible seal (60) and said lower portion of said support (20, 40).

9. The seal of claim 6, wherein said ring (94) is integrally formed with an inwardly-extending flange (24) of said lower portion of said support (20, 40) so that said ring (94) is disposed upwardly and inwardly from said inwardly-extending flange (24).

10. The seal of claim 6, wherein said ring (92, 94) comprises a half round cross-section.

11. The seal of claim 6, wherein said ring (90, 92, 94) comprises a rigid material and wherein said seal (200, 202, 204) comprises a guard (80) having a plurality of slits for protecting said flexible seal (60).

12. A cannula head (110, 510) comprising:
- a housing (130) having a passageway extending therethrough; and
- a seal (200, 202, 204) of claim 1 disposed across said passageway.

13. The cannula head of claim 12, wherein said seal (200, 202, 204) is releasably attachable to said cannula head (110, 510).

14. A trocar system (10, 12) comprising:
- a cannula head (110, 510) comprising a housing (130) having a passageway extending therethrough;
- a seal (200, 202, 204) of claim 1 disposed across said passageway;
- an obturator (400, 700) receivable in said cannula head (110, 510) and through said seal; and
- a cannula tube (300, 600) attachable to said cannula head (110, 510).

15. The trocar system of claim 14, wherein said seal (200, 202, 204) is releasably attachable to said cannula head (110, 510).

16. A method for sealing a cannula head (110, 510) with a seal according to claim 1, the method comprising:
- providing the support (20, 40) in the cannula head (110, 510) having a passageway extending therethrough defining an axis;
- providing the flexible seal (60)
- mounting the flexible seal (60) to the support (20; and across said passage way 40) in the cutout so that the flexible seal (60) is rotatable around the axis and the at least one of the upwardly-extending annular flange (72) and the downwardly-depending annular flange (74) is restrained by the cutout from lateral movement.

17. The method of claim 16 further providing the cutout in the support (20, 40), and wherein at least one of said upwardly-extending annular flange (72) and said downwardly-depending annular flange (74) of the flexible seal (60) is sized smaller that the cutout.

18. The method of claim 16, wherein said at least one of said upwardly-extending annular flange (72) and said downwardly-depending annular flange (74) is disposed in said cutout and spaced apart from a top surface, side surfaces, and a bottom surface of the cutout.

19. The method of claim 16 further comprising positioning a ring (90, 92, 94) having an opening sized smaller than a lower opening in the support between the support and the flexible seal.

20. The method of claim 16 further comprising releasably attaching the seal (200, 202, 204) to the cannula head (110, 510).

21. The method of claim 16 further comprising positioning a ring (90, 92, 94) within the central opening of the support (20, 40) between a lower opening of the support (20, 40) and the flexible seal (60), the ring (90, 92, 94) being larger in size than the lower opening and having an inner opening sized smaller than the lower opening of the support (20, 40), wherein at least one of said ring (90, 92) is movably positioned between said flexible seal (60) and said support (20, 40), said ring (94) being integrally formed as one piece with an inwardly-extending flange (24) of said lower portion of said support (20, 40) so that said ring (94) is disposed upwardly and inwardly from said inwardly-extending flange (24), and said ring (92, 94) comprising a half round cross-section.

## Patentansprüche

1. Dichtung (200, 202, 204) für einen Kanülenkopf (110, 510), wobei die Dichtung (200, 202, 204) Folgendes aufweist:
- einen Träger (20, 40), der einen durch ihn verlaufenden Durchgang bildet und eine Achse definiert;
- eine flexible Dichtung (60), die von dem Träger (20, 40) quer über den Durchgang drehbar gehalten ist, wobei die flexible Dichtung (60) einen oberen Teil (70), der einen horizontal verlaufenden Abschnitt (71) hat, und mindestens einen nach oben verlaufenden ringförmigen Flansch (72) oder/und einen nach unten herabhängenden ringförmigen Flansch (74) aufweist; wobei
der Träger (20, 40) einen Ausschnitt zur Aufnahme des oberen Teils (70) der flexiblen Dichtung (60) aufweist, wobei die Flansche (72, 74) und ein Teil des horizontal verlaufenden Abschnitts (71) in dem Ausschnitt angeordnet und von Oberflächen des Ausschnitts beabstandet sind, so dass eine seitliche Bewegung der Flansche (72, 74) eingeschränkt ist;
**dadurch gekennzeichnet, dass** die flexible Dichtung (60) von dem Träger (20, 40) so drehbar gehalten ist, dass die flexible Dichtung nur um die Achse drehbar ist.

2. Dichtung nach Anspruch 1, bei welcher die Dichtung (200, 202, 204) eine Schutzeinrichtung (80) aufweist, die eine Vielzahl von Schlitzen zum Schutz der flexiblen Dichtung (60) hat, und bei welcher die flexible Dichtung (60) eine Schmiermittelbeschichtung aufweist.

3. Dichtung nach Anspruch 1, bei welcher die Dichtung (200, 202, 204) am Kanülenkopf (110, 510) abnehmbar angebracht werden kann.

4. Dichtung nach Anspruch 1, bei welcher mindestens einer der Träger (20, 40) einen von dem Träger nach außen verlaufenden Arm (26) aufweist.

5. Dichtung nach Anspruch 4, bei welcher der Arm (26) einen nach oben verlaufenden Knopf aufweist.

6. Dichtung nach Anspruch 1, welche ferner einen Ring (90, 92, 94) aufweist, der zwischen einem unteren Abschnitt des Trägers (20, 40) und der flexiblen Dichtung (60) angeordnet ist, und bei welcher der Ring (90, 92, 94) eine Öffnung bildet, die kleiner dimensioniert ist als eine Öffnung in dem unteren Abschnitt des Trägers (20, 40).

7. Dichtung nach Anspruch 6, bei welcher der Ring (90, 92) zwischen der flexiblen Dichtung (60) und dem unteren Abschnitt des Trägers (20, 40) bewegbar ist.

8. Dichtung nach Anspruch 6, bei welcher der Ring (90, 92) zwischen der flexiblen Dichtung (60) und dem unteren Abschnitt des Trägers (20, 40) beweglich positioniert ist.

9. Dichtung nach Anspruch 6, bei welcher der Ring (94) mit einem nach innen verlaufenden Flansch (24) des unteren Abschnitts des Trägers (20, 40) einstückig gebildet ist, so dass der Ring (94) oberhalb und innerhalb des nach innen verlaufenden Flansches (24) angeordnet ist.

10. Dichtung nach Anspruch 6, bei welcher der Ring (92, 94) einen halbrunden Querschnitt aufweist.

11. Dichtung nach Anspruch 6, bei welcher der Ring (90, 92, 94) ein starres Material aufweist und bei welcher die Dichtung (200, 202, 204) eine Schutzeinrichtung (80) aufweist, die eine Vielzahl von Schlitzen zum Schutz der flexiblen Dichtung (60) hat.

12. Kanülenkopf (110, 510), enthaltend:
- ein Gehäuse (130), das einen durch dieses verlaufenden Durchgang hat; und
- eine Dichtung (200, 202, 204) nach Anspruch 1, die quer über den Durchgang angeordnet ist.

13. Kanülenkopf nach Anspruch 12, bei welcher die Dichtung (200, 202, 204) an dem Kanülenkopf (110, 510) abnehmbar anbringbar ist.

14. Trokarsystem (10, 12), enthaltend:
- einen Kanülenkopf (110, 510), der ein Gehäuse (130) mit einem durch dieses verlaufenden Durchgang aufweist;
- eine Dichtung (200, 202, 204) nach Anspruch 1, die quer über den Durchgang angeordnet ist;
- einen Obturator (400, 700), der in dem Kanülenkopf (110, 510) und durch die Dichtung aufgenommen werden kann; und
- ein Kanülenrohr (300, 600), das an dem Kanülenkopf (110, 510) anbringbar ist.

15. Trokarsystem nach Anspruch 14, bei welchem die Dichtung (200, 202, 204) an dem Kanülenkopf (110, 510) abnehmbar anbringbar ist.

16. Verfahren zur Abdichtung eines Kanülenkopfes (110, 510) mit einer Dichtung nach Anspruch 1, welches Verfahren enthält:
- Bereitstellen des Trägers (20, 40) in dem Kanülenkopf (110, 510), der einen durch diesen verlaufenden Durchgang aufweist, der eine Achse definiert;
- Bereitstellen der flexiblen Dichtung (60) quer über den Durchgang; und
- Anbringen der flexiblen Dichtung (60) an dem Träger (20, 40) in dem Ausschnitt, so dass die flexible Dichtung (60) um die Achse drehbar ist und der mindestens eine des nach oben verlaufenden ringförmigen Flansches (72) und des nach unten herabhängenden ringförmigen Flansches (74) durch den Ausschnitt gegen seitliche Bewegung festgehalten wird.

17. Verfahren nach Anspruch 16, welches ferner den Ausschnitt in dem Träger (20, 40) bereitstellt, und bei welchem mindestens der nach oben verlaufende ringförmige Flansch (72) und/oder der nach unten herabhängende ringförmige Flansch (74) der flexiblen Dichtung (60) kleiner dimensioniert ist als der Ausschnitt.

18. Verfahren nach Anspruch 16, bei welchem mindestens einer des nach oben verlaufenden ringförmigen Flansches (72) und des nach unten herabhängenden ringförmigen Flansches (74) in dem Ausschnitt angeordnet ist und von einer oberen Oberfläche, Seitenoberflächen und einer unteren Oberfläche des Ausschnitts beabstandet ist.

19. Verfahren nach Anspruch 16, ferner enthaltend das Positionieren eines Ringes (90, 92, 94) mit einer Öffnung, die kleiner dimensioniert ist als eine untere Öffnung in dem Träger, zwischen dem Träger und der flexiblen Dichtung.

20. Verfahren nach Anspruch 16, ferner enthaltend das entfernbare Anbringen der Dichtung (200, 202, 204) an dem Kanülenkopf (110, 510).

21. Verfahren nach Anspruch 16, ferner enthaltend das Positionieren eines Ringes (90, 92, 94) innerhalb der zentralen Öffnung des Trägers (20, 40) zwischen einer unteren Öffnung des Trägers (20, 40) und der flexiblen Dichtung (60), welcher Ring (90, 92, 94) eine größere Abmessung hat als die untere Öffnung und eine innere Öffnung hat, die kleiner dimensioniert ist als die untere Öffnung des Trägers (20, 40), wobei mindestens einer der Ringe (90, 92) zwischen der flexiblen Dichtung (60) und dem Träger (20, 40) beweglich positioniert ist, welcher Ring (94) integriert als ein Stück mit einem nach innen verlaufenden Flansch (24) des unteren Abschnitts des Trägers (20, 40) gebildet ist, so dass der Ring (94) oberhalb und innerhalb des nach innen verlaufenden Flansches (24) angeordnet ist, und welcher Ring (92, 94) einen halbrunden Querschnitt hat.

## Revendications

1. Joint (200, 202, 204) pour tête de canule (110, 510), le joint (200, 202, 204) comprenant :
- un support (20, 40) définissant un passage s'étendant à travers celui-ci et définissant un axe ;
- un joint souple (60) supporté de manière rotative par le support (20, 40) le joint souple (60) comprenant une partie supérieure (70) ayant une partie s'étendant horizontalement (71), et au moins un rebord parmi un rebord annulaire s'étendant vers le haut (72) et un rebord annulaire suspendu vers le bas (74) ; dans lequel
le support (20, 40) comprend une découpe pour recevoir la partie supérieure (70) du joint souple (60), les rebords (72, 74) et une partie de la partie s'étendant horizontalement (71) étant disposés dans la découpe et écartés des surfaces de la découpe de sorte qu'un déplacement latéral des rebords (72, 74) est limité ;
**caractérisé en ce que** le joint souple (60) est supporté de manière rotative par le support (20, 40) de sorte que le joint souple peut tourner uniquement autour de l'axe.

2. Joint selon la revendication 1, dans lequel le joint (200, 202, 204) comprend une protection (80) ayant plusieurs fentes pour protéger le joint souple (60), et dans lequel le joint souple (60) comprend un revêtement lubrifiant.

3. Joint selon la revendication 1, dans lequel le joint (200, 202, 204) peut être fixé de manière libérable sur la tête de canule (110, 510).

4. Joint selon la revendication 1, dans lequel au moins un élément du support (20, 40) comprend un bras (26) s'étendant vers l'extérieur à partir du support.

5. Joint selon la revendication 4, dans lequel le bras (26) comprend un bouton s'étendant vers le haut.

6. Joint selon la revendication 1, dans lequel il comprend de plus un anneau (97, 92, 94) disposé entre une partie inférieure du support (20, 40) et le joint souple (60), et dans lequel l'anneau (90, 92, 94) définit une ouverture de dimension plus petite qu'une ouverture dans la partie inférieure du support (20, 40).

7. Joint selon la revendication 6, dans lequel l'anneau (90, 92) est mobile entre le joint souple (60) et la partie inférieure du support (20, 40).

8. Joint selon la revendication 6, dans lequel l'anneau (90, 92) est positionné de manière mobile entre le joint souple (60) et la partie inférieure du support (20, 40).

9. Joint selon la revendication 6, dans lequel l'anneau (94) est formé d'un seul tenant avec un rebord s'étendant vers l'intérieur (24) de la partie inférieure du support (20, 40) de sorte que l'anneau (94) est disposé vers le haut et vers l'intérieur à partir du rebord s'étendant vers l'intérieur (24).

10. Joint selon la revendication 6, dans lequel l'anneau (92, 94) a une section transversale en demi-rond.

11. Joint selon la revendication 6, dans lequel l'anneau (90, 92, 94) est constitué d'un matériau rigide et dans lequel le joint (200, 202, 204) comprend une protection (80) ayant plusieurs fentes pour protéger le joint souple (60).

12. Tête de canule (110, 510) comprenant :
- un boîtier (130) ayant un passage s'étendant à travers celui-ci ; et
- un joint (200, 202, 204) selon la revendication 1 disposé à travers le passage.

13. Tête de canule selon la revendication 12, dans lequel le joint (200, 202, 204) peut être relié de manière libérable à la tête de canule (110, 510).

14. Système de trocart (10, 12) comprenant :
- une tête de canule (110, 510) comprenant un boîtier (130) ayant un passage s'étendant à travers celui-ci ;
- un joint (200, 202, 204) selon la revendication 1 disposé à travers ledit passage ;
- un obturateur (400, 700) pouvant être reçu dans la tête de canule (110, 510) et à travers le joint ; et
- un tube de canule (300, 600) pouvant être fixé sur la tête de canule (110, 510).

15. Système de trocart selon la revendication 14, dans lequel le joint (200, 202, 204) peut être fixé de manière libérable sur la tête de canule (110, 510).

16. Procédé pour rendre étanche une tête de canule (110, 510) ayant un joint selon la revendication 1, le procédé consistant à :
- agencer le support (20, 40) dans la tête de canule (110, 510) ayant un passage s'étendant à travers celui-ci définissant un axe ;
- agencer le joint souple (60) ; et
- monter le joint souple (60) sur le support (20, 40) dans la découpe de sorte que le joint souple (60) soit rotatif autour de l'axe et le au moins un rebord parmi le rebord annulaire s'étendant vers le haut (72) et le rebord annulaire suspendu vers le bas (74) est retenu par la découpe vis-à-vis d'un déplacement latéral.

17. Procédé selon la revendication 16 fournissant de plus la découpe dans le support (20, 40), et dans lequel au moins un rebord parmi le rebord annulaire s'étendant vers le haut (72) et le rebord annulaire suspendu vers le bas (74) du joint souple (60) a une dimension plus petite que la découpe.

18. Procédé selon la revendication 16, dans lequel le au moins un rebord parmi le rebord annulaire s'étendant vers le haut (72) et le rebord annulaire suspendu vers le bas (74) est disposé dans la découpe et est écarté d'une surface supérieure, des surfaces latérales, et d'une surface de fond de la découpe.

19. Procédé selon la revendication 16, comprenant de plus le positionnement d'un anneau (90, 92, 94) ayant une ouverture de dimension plus petite qu'une ouverture inférieure située dans le support entre le support et le joint souple.

20. Procédé selon la revendication 16, comprenant de plus la fixation de manière libérable du joint (200, 202, 204) sur la tête de canule (110, 510).

21. Procédé selon la revendication 16 comprenant de plus le positionnement d'un anneau (90, 92, 94) dans l'ouverture centrale du support (20, 40) entre une ouverture inférieure du support (20, 40) et le joint souple (60), l'anneau (90, 92, 94) ayant une dimension plus grande que l'ouverture inférieure et ayant une ouverture intérieure de dimension plus petite que l'ouverture inférieure du support (20, 40), dans lequel au moins un élément de l'anneau (90, 92) est positionné de manière mobile entre le joint souple (60) et le support (20, 40), l'anneau (94) étant formé d'un seul tenant sous la forme d'une seule pièce avec un rebord s'étendant vers l'intérieur (24) de la partie inférieure du support (20, 40) de sorte que l'anneau (94) est disposé vers le haut et vers l'intérieur à partir du rebord s'étendant vers l'intérieur (24), et l'anneau (92, 94) comprenant une section transversale en demi-rond.
